(19) Europäisches Patentamt

European Patent Office

Office européen des brevets

(11) **EP 0 868 910 B1**

(12) **EUROPÄISCHE PATENTSCHRIFT**

(45) Veröffentlichungstag und Bekanntmachung des Hinweises auf die Patenterteilung:
**02.01.2003 Patentblatt 2003/01**

(51) Int Cl.7: **A61K 9/20**

(21) Anmeldenummer: **98103626.2**

(22) Anmeldetag: **02.03.1998**

(54) **Verwendung von redispergierbaren Polymerpulvern oder Polymergranulaten als Bindemittel zur Herstellung von festen pharmazeutischen Darreichungsformen**

Use of redispersible polymer powders or polymer granulates as binders for the preparation of solid pharmaceutical delivery forms

Utilisation de poudres polymères redispersibles comme liants pour la préparation de formes d'administration pharmaceutiques solides

(84) Benannte Vertragsstaaten:
**AT BE CH DE DK ES FI FR GB GR IE IT LI LU NL PT SE**
Benannte Erstreckungsstaaten:
**RO SI**

(30) Priorität: **10.03.1997 DE 19709663**

(43) Veröffentlichungstag der Anmeldung:
**07.10.1998 Patentblatt 1998/41**

(73) Patentinhaber: **BASF AKTIENGESELLSCHAFT 67056 Ludwigshafen (DE)**

(72) Erfinder:
- **Kolter, Karl, Dr.**
  **67117 Limburgerhof (DE)**
- **Tiefensee, Kristin, Dr.**
  **67368 Westheim (DE)**
- **Zeitz, Katrin, Dr.**
  **67067 Ludwigshafen (DE)**

(56) Entgegenhaltungen:
EP-A- 0 231 826      DE-A- 3 810 343
DE-C- 4 341 156

## Beschreibung

Die vorliegende Erfindung betrifft die Verwendung von redispergierbaren Polymerpulvern oder Polymergranulaten bestehend aus Polyvinylacetat und N-Vinylpyrrolidon-haltigen Polymeren als Bindemittel zur Herstellung von festen pharmazeutischen Darreichungsformen.

Um Pharmaka ihrem Wirkort zuführen zu können, ist es notwendig, sie in eine Form zu bringen, die den physiologischen Gegebenheiten des Applikationsorts und den physikalisch-chemischen Eigenschaften des Wirkstoffs gerecht wird. Nur in seltenen Fällen ist eine Verabreichung ohne jegliche Formung, d.h. als Einzeldosis, die nur den Wirkstoff enthält, möglich. In der Regel wird die Überführung der Wirkstoffe in geeignete Arzneiformen erst durch die Verwendung von Hilfsstoffen ermöglicht.

Sowohl Wirk- als auch Hilfsstoffe, wie beispielsweise Füllstoffe, verfügen jedoch häufig über ein sehr schlechtes Bindevermögen, so daß der Einsatz von zusätzlichen Bindemitteln zur Herstellung einer festen Darreichungsform unumgänglich ist. Die Menge und Art des Bindemittels, aber auch das Verarbeitungsverfahren beeinflussen maßgeblich die Eigenschaften fester Darreichungsformen, z.B. die eines Granulates, wie Korngröße, Fließfähigkeit, Verpreßbarkeit, Staubneigung, Porosität oder Oberflächenstruktur sowie die Eigenschaften daraus hergestellter Komprimate, wie beispielsweise Bruchfestigkeit, Friabilität, Zerfall und Freisetzung des Wirkstoffs.

In der pharmazeutischen Anwendungstechnik unterscheidet man je nach Verarbeitungsverfahren zwischen Feucht- und Trockenbindemittel. Letztere werden u.a. bei der Direkttablettierung und bei der Trockengranulation bzw. Kompaktierung verwendet. Hierbei wird das Bindemittel mit dem Wirkstoff und gegebenenfalls weiteren Hilfsstoffen vermischt und anschließend direkttablettiert oder granuliert bzw. kompaktiert. Trockenbindemittel müssen eine gewisse Plastizität unter Druck aufweisen, da dadurch die Kontaktflächen zwischen Bindemittel und Wirkstoff- bzw. Hilfsstoffteilchen vergrößert werden, so daß die Haftkräfte zunehmen und als Folge ein festeres Granulat bzw. eine festere Tablette resultiert.

Im Gegensatz dazu wird bei der Feuchtgranulation die Wirkstoff-Hilfsstoffmischung mit einer Lösung des Bindemittels in Wasser oder einem organischen Lösungsmittel befeuchtet, die feuchte Masse durch ein Sieb gegeben und anschließend getrocknet. Befeuchtung und Trocknung können dabei auch parallel ablaufen, wie z. B. in der Wirbelschichtgranulation.

Für eine optimale Verarbeitung soll das Bindemittel in Lösung niedrigviskos sein, da viskose Lösungen zu inhomogenen Granulaten führen.

Aufgrund der Tatsache, daß der Zerfall der Arzneiformen sowie die Freisetzungsgeschwindigkeit der Wirkstoffe durch das Bindemittel nicht nennenswert beeinflußt werden sollen, werden im Markt derzeit für Instant-Release-Formen nur wasserlösliche Bindemittel verwendet.

Die gängigsten Bindemittel sind beispielsweise Polyvinylpyrrolidon, Vinylacetat-Vinylpyrrolidon Copolymere, Gelatine, Stärkekleister, Maltodextrine, hydroxyalkylierte bzw. carboxyalkylierte Cellulosederivate, wie Hydroxypropylmethylcellulose, Methylcellulose, Natriumcarboxymethylcellulose sowie natürliche Gummisorten, wie beispielsweise Gummi Arabicum, Pektin oder Alginat.

Viele dieser Bindemittel weisen in Lösung eine hohe Viskosität auf und sind schlecht verarbeitbar. All diesen Bindemitteln mangelt es an der gewünschten Plastizität, die in der Feuchtgranulation, besonders aber in der Direkttablettierung oder Kompaktierung eine Rolle spielt. Je höher die Plastizität des Bindemittels, desto mechanisch stabiler sind die daraus hergestellten Granulate und Tabletten.

In dem US-Patent 5,252,704 wird die Herstellung von redispergierbaren Polymerpulvern unter Verwendung von Polyvinylpyrrolidon als Dispergierhilfsstoff beschrieben. Als Einsatzgebiet ist die Anwendung in der Zementherstellung angegeben. Pharmazeutische Anwendungen hingegen sind nicht erwähnt.

DE-B-4341156 beschreibt die Verwendung von in Wasser dispergierbaren Kunststoffdispersionspulvern mit einer Kern-Hülle-Struktur als Arzneimittelträger in Arzneiformen mit retardierter Wirkstoff-Freisetzung, wobei der Anteil an Dispersionspulver in der Tablette mehr als 75 Gew.-% ausmacht.

In DE-A-4220782 werden Verfahren zur Herstellung von festen pharmazeutischen Retardformen durch Auftragen eines Bindemittels auf einen wirkstoffhaltigen Kern beschrieben. Bei den hier verwendeten Bindemitteln handelt es sich um Polymere auf (Meth)acrylatbasis.

Die EP-A-0231826 offenbart eine Tablettenformulierung zur verlängerten Freisetzung, welche als Hilfsstoffe Polyvinylacetat und Polyvinylpyrrolidon enthält.

Der vorliegenden Erfindung lag die Aufgabe zugrunde, Polymerpulver oder Polymergranulate, bestehend aus einem wasserunlöslichen und wasserlöslichen Polymer zu finden, die als Bindemittel zur Herstellung von festen pharmazeutischen Darreichungsformen geeignet sind, wobei der Anteil des Bindemittels in der Darreichungsform weniger als 20 Gew.-% betragen sollte. Die auf diese Weise hergestellten Darreichungsformen sollten darüberhinaus eine schnelle Freisetzung der Wirkstoffe ermöglichen.

Die Aufgabe wurde erfindungsgemäß gelöst durch Verwendung von redispergierbaren Polymerpulvern oder Polymergranulaten aus

a) 10 bis 95 Gew.-% Polyvinylacetat,

b) 5 bis 90 Gew.-% eines N-Vinylpyrrolidon-haltigen Polymers,

c) 0 bis 20 Gew.-% eines weiteren wasserlöslichen oder wasserquellbaren Stoffes und

d) 0 bis 20 Gew.-% eines wasserunlöslichen Puderungsmittels sowie gegebenenfalls

e) weiteren Zusatzstoffen,

als Bindemittel zur Herstellung von festen pharmazeutischen Darreichungsformen, wobei der Anteil des Bindemittels in der Darreichungsform 0,5 bis 20 Gew.-% beträgt.

Überraschenderweise wurde nun gefunden, daß Polymerpulver oder Polymergranulate aus Polyvinylacetat und N-Vinylpyrrolidon-haltigen Polymeren, insbesondere Polyvinylpyrrolidon und Vinylacetat-Vinylpyrrolidon Copolymeren oder Mischungen davon, über ausgezeichnete Bindemittelwirkungen verfügen und zudem in Konzentrationsbereichen von 0,5 bis 20 Gew.-% der Gesamtmenge der Formulierung den Zerfall bzw. die Wirkstoff-Freisetzung nicht nennenswert beeinflussen. Letzteres wäre aufgrund des Einbringens eines wasserunlöslichen Polymeren wie Polyvinylacetat zu erwarten gewesen.

Beide Polymere ergänzen sich in idealer Weise. Polyvinylpyrrolidon verfügt über eine ausgezeichnete Wasserlöslichkeit sowie über sehr gutes Hydrophilisierungs- und Stabilisierungsvermögen für Wirkstoffe und verbessert die Auflösung von Wirkstoffen in Körperflüssigkeiten. Polyvinylacetat ist wasserunlöslich und war als Bindemittel bis dato nicht beschrieben. Erst durch die Kombination mit Polyvinylpyrrolidon bzw. mit Vinylacetat-Vinylpyrrolidon Copolymeren konnte diese Anwendung erschlossen werden. Polyvinylacetat erhöht deutlich die Plastizität im Endprodukt. Beide Polymere sind - wie DSC-Untersuchungen belegen - nicht miteinander mischbar, so daß eine einfache Kombination beispielsweise durch eine homogene Schmelze nicht möglich ist. Zudem wurde gefunden, daß für eine gute Bindemittelwirksamkeit das Polyvinylacetat im Produkt sehr fein verteilt sein muß.

Die Herstellung der redispergierbaren Polymerpulver erfolgt in der Weise, daß zunächst Vinylacetat emulsionspolymerisiert wird und zu der entstandenen scherstabilen und feinteiligen Dispersion das N-Vinylpyrrolidon-haltige Polymer sowie gegebenenfalls weitere Hilfsstoffe zugegeben werden und die Mischung sprühgetrocknet wird. Bevorzugt eingesetzte N-Vinylpyrrolidon-haltige Polymere sind beispielsweise Polyvinylpyrrolidon und Vinylacetat-Vinylpyrrolidon Copolymere.

Die K-Werte der Polymerisate sollen im Bereich von 10 bis 350, vorzugsweise 30 bis 150, besonders bevorzugt im Bereich von 50 und 90 liegen. Der jeweils gewünschte K-Wert läßt sich in an sich bekannter Weise durch die Wahl der Polymerisationsbedingungen, beispielsweise der Polymerisationsdauer und der Initiatorkonzentration einstellen. Die K-Werte werden nach Fikentscher, Cellulosechemie, Bd. 13, S. 58-64 und 71-74 (1932) bei 25°C in 0,1 Gew.-%iger wäßriger Lösung gemessen.

Der Zusatz von feinteiligen, wasserunlöslichen Sprühhilfsmitteln bei der Sprühtrocknung kann ein Verkleben der gebildeten Teilchen verhindern. Hierbei ist ein Einstäuben dieser Sprühhilfsmittel in den Sprühtrockner besonders vorteilhaft. Es ist aber auch möglich, die Sprühhilfsmittel bereits der zu versprühenden Dispersion zuzusetzen oder sie erst nach der Sprühtrocknung zur Verhinderung eines Zusammenbackens der Teilchen unterzumischen. Als Puderungsmittel können bis zu 20 Gew.-%, bezogen auf den Feststoffgehalt, feinteilige wasserunlösliche Stoffe, insbesondere mindestens ein Vertreter aus der Gruppe Cellulose, bevorzugt mikrokristalline Cellulose, disperse Kieselsäure, Talkum, Bentonit, Magnesiumstearat oder ein Calciumphosphat verwendet werden.

Die Emulsionspolymerisation wird in bekannter Weise bei Temperaturen von 50°C bis 95°C, bevorzugt von 60°C bis 80°C, in Gegenwart von bekannten Polymerisationsinitiatoren durchgeführt.

Als Polymerisationsinitiatoren kommen vorzugsweise Radikalbildner, beispielsweise Peroxide wie bevorzugt Peroxosulfate, Peroxodisulfate und Azoverbindungen wie Azodiisobutyronitril in Betracht.

Als Emulgatoren können sowohl ionische wie nicht-ionische Emulgatoren oder deren Mischungen eingesetzt werden. Ihre Gesamtkonzentration liegt zwischen 0,2 und 10 Gew.-%, bevorzugt zwischen 0,4 und 7 Gew.-%, bezogen auf den Gesamtmonomerengehalt. Ferner können als weitere Hilfsstoffe bis zu 20 Gew.-%, bezogen auf den Feststoffgehalt, wasserlösliche oder wasserquellbare Schutzkolloide eingesetzt werden, wie z.B. Cellulosederivate, bevorzugt Hydroxypropylmethylcellulose, Methylcellulose oder Hydroxyethylcellulose, Galactomannan, Pectin, Xanthan, Polyvinylalkohol, Acrylat-Methacrylat Copolymere, Natriumcarboxymethylstärke, Cellulose, abgebaute Stärken, Maltodextrine etc. Die emulgierenden Hilfsstoffe können dabei vor, während und nach der Polymerisation zugegeben werden.

Die Dispersion weist einen Feststoffgehalt von 10 bis 45 Gew.-%, bevorzugt von 15 bis 35 Gew.-% auf.

Die sedimentationsstabile Polymerdispersion weist mittlere Teilchengrößen von 0,1 bis 7 µm, bevorzugt von 0,3 bis 4 µm auf. Die Bestimmung erfolgt in der üblichen Weise z.B. mittels Ultrazentrifuge, Photonenkorrelationsspektroskopie oder durch Bestimmung der Lichtdurchlässigkeit. Die Partikelgröße wird üblicherweise über die Emulgatorkonzentration bzw. die Temperaturführung gesteuert.

Eine entscheidende Bedeutung kommt der Scherstabilität der Polyvinylacetat-Dispersion zu. Nur sehr scherstabile Dispersionen sind in der Lage, nach dem Versprühen ausreichend redispergierbare Pulver zu bilden.

Die Prüfung der Scherstabilität erfolgt durch Scherung der Dispersion mittels eines Noppenrührers bei 2000 U/min über 15 min und gravimetrische Bestimmung des Koagulates nach Siebung über ein 125 um Sieb. Scherstabile Dispersionen weisen Koagulatanteile < 0,1% auf.

Die Zugabe des Polyvinylpyrrolidons erfolgt vorzugsweise als 10 bis 50 Gew.-%ige Lösung unter ständigem Rühren. Bei der Zugabe als Feststoff oder als höherkonzentrierte Lösung können Koagulationen auftreten.

Die Sprühtrocknung erfolgt in üblicher Weise in Sprühtürmen, wobei die zu trocknende Dispersion mittels Düsen oder Scheiben zerstäubt wird. Die Zuführung des heißen Gases, z. B. Luft oder Stickstoff, kann im Gleichstrom oder im Gegenstrom erfolgen, wobei das Gleichstromverfahren besonders bevorzugt ist, da es zu einer geringeren Temperaturbelastung führt.

Auch die FSD-Technologie (Fluidized Spray Drying), bei der zunächst sprühgetrocknet und sofort anschließend in der Wirbelschicht agglomeriert wird, kann eingesetzt werden. Alternativ kann auch gefriergetrocknet werden.

Das Gewichtsverhältnis von Polyvinylacetat zu Polyvinylpyrrolidon bzw. Vinylacetat-Vinylpyrrolidon Copolymeren kann im Verhältnis 95:5 bis 10:90 variiert werden. Besonders bevorzugt sind Verhältnisse zwischen 90:10 und 30:70. Darüber hinaus können die Produkte weitere hydrophile, wasserlösliche Polymere, wie z. B. Polyvinylalkohol, Hydroxypropylmethylcellulose, Hydroxypropylcellulose, Methylcellulose, Hydroxyethylcellulose, Galactomannan, Pectin, Xanthan, Acrylat-Methacrylat Copolymere, Natriumcarboxymethylstärke bzw. -cellulose, abgebaute Stärken, Maltodextrine oder auch niedermolekulare Stoffe, wie z. B. Monosaccharide, Disaccharide, Zuckeralkohole, anorganische wasserlösliche Salze, Aminosäuren, wasserlösliche Säuren bzw. deren Salze oder Tenside enthalten. Ein Zusatz dieser hydrophilen Hilfsstoffe kann auch nach der Redispergierung eines Produktes bestehend aus Polyvinylacetat und Polyvinylpyrrolidon bzw. Vinylacetat-Vinylpyrrolidon Copolymeren zur Bindemitteldispersion erfolgen. Diese hydrophilen Hilfsstoffe können auf die Polyvinylacetatdispersionen einen zusätzlichen stabilisierenden Effekt ausüben und förderlich für eine schnelle Freisetzung des Wirkstoffes aus der Darreichungsform sein. Besonders bevorzugt ist die Verwendung von Polyvinylalkohol und Methylhydroxypropylcellulose.

Zusätzliche hydrophile Eigenschaften der erfindungsgemäßen, redispergierbaren Polymerpulver können durch Verwendung von teilweise hydrolysiertem Polyvinylacetat eingestellt werden, wobei hier insbesondere Polyvinylacetate mit einem Hydrolysegrad von bis zu 50 Mol-% verwendet werden.

Die erfindungsgemäßen Polymerpulver oder Polymergranulate weisen in der Granulation und der Tablettierung sowohl Trocken- als auch Feuchtbindemittelwirksamkeiten auf, die herkömmliche Bindemittel deutlich übertreffen. Dies läßt sich anhand von höheren Bruchfestigkeiten und niedrigeren Friabilitäten belegen. Die Zerfalls-und Freisetzungseigenschaften von, mit den erfindungsgemäßen Produkten hergestellten pharmazeutischen Zubereitungen, unterscheiden sich nicht von vergleichbaren Zubereitungen mit herkömmlichen Bindemitteln. Bei der Feuchtgranulation erweist sich die niedrige Viskosität in Wasser als besonderer Vorteil, da diese höhere Bindemittelkonzentration erlaubt. Dadurch lassen sich Granulierzeiten und Granulierkosten reduzieren.

Bei der Anwendung als Feuchtbindemittel wird das erfindungsgemäße Produkt unter Rühren in Wasser eingetragen, wobei üblicherweise Konzentrationen von 5 bis 30 Gew.-% eingestellt werden. Anschließend können die beschriebenen hydrophilen Hilfsstoffe ebenfalls unter Rühren zugegeben werden. Nach kurzer Zeit ist die Dispersion gebrauchsfertig und kann zu Granulationszwecken verwendet werden. Die Konzentrationen des Bindemittels im Endprodukt liegen vorzugsweise zwischen 1 und 10 Gew.-%.

Bei der Anwendung als Trockenbindemittel wird das erfindungsgemäße Produkt mit dem Wirkstoff und weiteren Hilfsstoffen, beispielsweise Füllstoffen, Fließhilfsmitteln, Sprengmitteln und Gleitmitteln trocken gemischt und verpreßt oder kompaktiert. Üblicherweise werden 1 bis 15 Gew.-% an erfindungsgemäßem Polymergemisch verwendet. Es wurde nachgewiesen, daß die Trockenbindewirksamkeit mit steigender Konzentration des erfindungsgemäßen Produktes in der Darreichungsform steigt.

Bei den oben genannten Darreichungsformen, die die erfindungsgemäßen polymeren Bindemittel enthalten, handelt es sich bevorzugt um feste Zubereitungen. Darunter versteht man u.a. Tabletten, Mikrotabletten, Dragees, Pastillen, Kapseln, Granulate oder Pellets.


Beispiel 1


Herstellung eines redispergierbaren Pulvers aus ca. 95 Gew.-% Polyvinylacetat/Polyvinylpyrrolidon im Gewichtsverhältnis 80:20 und 5 Gew.-% Polyvinylalkohol.

In einem Rührgefäß mit Rückflußkühler wurden 500 g Vinylacetat mit 0,5 Gew.-% Natriumlaurylsulfat unter Stickstoff in Wasser emulgiert und durch Initiierung mit 0,3 Gew.-% Natriumpersulfat bei 75 °C polymerisiert. Nach Zugabe von 5 Gew.-% Polyvinylalkohol (Mowiol® 8-88 der Fa. Hoechst) wurde das Reaktionsgemisch auf Raumtemperatur abgekühlt. Man erhielt eine Dispersion mit einem Feststoffgehalt von 27 Gew.-% und einer mittleren Teilchengröße von 1 mm.

Die Dispersion wurde mit 417 g einer 30 Gew.-%igen wäßrigen Polyvinylpyrrolidon-Lösung (Kollidon® K 30 der Fa. BASF) versetzt und die Mischung im Stickstoffgleichstrom bei einer Eingangstemperatur von 140 °C sprühgetrocknet. Man erhielt ein weißes, frei fließendes Pulver.

Beispiel 2

Ermittlung der Preßeigenschaften von Polyvinylacetat/Polyvinylpyrrolidon im Gewichtsverhältnis 80:20 (K-Wert: 30) im Vergleich zu Polyvinylpyrrolidon (K-Wert: 30).

Auf einer Korsch Excenterpresse EKO wurde eine Mischung aus dem jeweiligen Bindemittel und 0,5 Gew.-% Magnesiumstearat, die in einem Turbula-Mischer mit einer Mischzeit von 10 Minuten hergestellt wurde, zu Tabletten mit einem Durchmesser von 12 mm und 300 mg Gewicht verpreßt. Die Tablettenpresse war voll instrumentiert, so daß Kraft-Zeit- und Kraft-Weg-Kurven registriert werden konnten. Aus diesen Daten wurden weitere Verpressungsparameter über ein Softwareprogramm berechnet.

| | Polymergemisch aus 80 Gew.-% Polyvinylacetat und 20 Gew.-% Polyvinylpyrrolidon | Polyvinylpyrrolidon (K-Wert 30) |
|---|---|---|
| Preßdruck MPa | 162 MPa | 160 MPa |
| Verdichtungs widerstand | 3,0 | 3,8 |
| Tensile Strength [MPa] | 7,41 MPa | 2,44 MPa |

Der Verdichtungswiderstand R ist definiert als

$$R = \Delta \text{ lg Preßdruck} / \Delta \text{ lg Scheindichte}.$$

Je niedriger der R-Wert, desto weniger Widerstand setzt das Material der Verpressung entgegen.

Die Tensile Strength ist definiert als

$$TS = 2 \text{ x Bruchfestigkeit} / \pi \text{ x Durchmesser x Höhe}.$$

Höhere Tensile Strength-Werte sind charakteristisch für bessere mechanische Eigenschaften.

Es zeigte sich, daß das Gemisch Polyvinylacetat/Polyvinylpyrrolidon im Gewichtsverhältnis 80:20 leichter verpreßbar war als Polyvinylpyrrolidon und zu wesentlich höheren Festigkeiten führte.

Beispiel 3

Verwendung von Polyvinylacetat/Polyvinylpyrrolidon im Gewichtsverhältnis 80:20 als Trockenbindemittel in einer direkttablettierten Ascorbinsäure-Tablette

| Rezeptur: | |
|---|---|
| Ascorbinsäure krist. | 200,0 mg |
| Ludipress® (Fa. BASF) | 237,5 mg |
| Trockenbindemittel, bestehend aus 80 Gew.-% Polyvinylacetat und 20 Gew.-% Polyvinylpyrrolidon | 50,0 mg |
| Kollidon® CL (Fa. BASF) | 10,0 mg |
| Magnesiumstearat | 2,5 mg |
| Gesamtgewicht | 500,0 mg |

Alle Inhaltsstoffe wurden über ein 0,8 mm-Sieb gegeben, im Turbula-Mischer 10 Minuten gemischt und anschließend auf einer voll instrumentierten Korsch Excenterpresse EKO bei einer Preßkraft von 18 kN zu biplanen, facettierten Tabletten mit 12 mm Durchmesser und 500 mg Gewicht verpreßt. Die Mischung ließ sich ausgezeichnet verpressen und führte zu Tabletten mit folgenden Eigenschaften:

| Bruchfestigkeit: | 93 N |
| Friabilität: | < 0,2 % |
| Zerfall: | 1 min |
| Freisetzung: (in 900 ml künstl. Magensaft, 100 U/min) | 98,8 % nach 30 min |

Wurde anstelle der Polyvinylacetat-Polyvinylpyrrolidon-Kombination Maltodextrin als Bindemittel in der gleichen Menge verwendet, so lag die Bruchfestigkeit bei nur 53 N, im Falle von reinem Polyvinylpyrrolidon (Kollidon® K 30, Fa. BASF) bei 68 N und im Falle von einem Copolymer aus Vinylacetat und Vinylpyrrolidon (Kollidon® VA 64, Fa. BASF) bei 79 N.

Beispiel 4

Verwendung einer Polymermischung aus 8 Gew.-Teilen Polyvinylacetat und 2 Gew.-Teilen Vinylpyrrolidon-Vinylacetat Copolymer (40:60) und 5 Gew.-% Polyvinylalkohol als Trockenbindemittel in einer direkttablettierten Ibuprofen-Tablette

| Rezeptur: | |
| --- | --- |
| Ibuprofen | 400,0 mg |
| mikrokristalline Cellulose (Avicel® PH 102) | 133,0 mg |
| Trockenbindemittel, bestehend aus 8 Gew.-Teilen Polyvinylacetat und 2 Gew.-Teilen Vinylpyrrolidon-Vinylacetat Copolymer (40:60) und 5 Gew.-% Polyvinylalkohol | 40,0 mg |
| Kollidon® CL | 18,0 mg |
| Aerosil® 200 | 6,0 mg |
| Magnesiumstearat | 3,0 mg |
| Gesamtgewicht | 600,0 mg |

Alle Inhaltsstoffe außer Magnesiumstearat wurden über ein 0,8 mm-Sieb gegeben, in einem Lödige-Mischer 1 Minute gemischt, mit dem ebenfalls gesiebten Magnesiumstearat versetzt und nochmals 1 Minute gemischt. Diese Tablettiermischung (Ansatzgröße: 1,8 kg) wurde auf einer Korsch Excenterpresse EKO bei 80 MPa Preßdruck zu biplanen, facettierten Tabletten mit 12 mm Durchmesser und 600 mg Gewicht verpreßt.

| Eigenschaften der Tabletten: | |
| --- | --- |
| Tensile Strength: | 1,5 MPa |
| Friabilität: | 0,1 % |
| Zerfall: | 1 min |
| Freisetzung: (Methode nach USP) | 99,5 % nach 30 min |

Beispiel 5

Verwendung einer Polymermischung aus 99 Gew.-% Polyvinylacetat/Polyvinylpyrrolidon im Gewichtsverhältnis 50:50 und 1 Gew.-% hochdisperse Kieselsäure als Feuchtbindemittel in einer Paracetamol-Tablette

| Rezeptur: | |
| --- | --- |
| Paracetamol Pulver | 150,0 mg |
| Lactose EP D 20 | 155,0 mg |

(fortgesetzt)

| Rezeptur: | |
|---|---|
| Maisstärke | 155,0 mg |
| Bindemittel, bestehend aus 99 Gew.-% Polyvinylacetat/Polyvinylpyrrolidon im Gewichtsverhältnis 50: 50 und 1 Gew.-% hochdisperse Kieselsäure | 15,0 mg |
| Kollidon® CL | 22,5 mg |
| Magnesiumstearat | 2,5 mg |
| Gesamtgewicht | 500,0 mg |

3,0 kg Paracetamol Pulver, 3,10 kg Lactose EP D 20 und 3,10 kg Maisstärke wurden über ein 0,8 mm-Sieb gesiebt und in einem Diosna-Mischer 1 Minute gemischt. 0,3 kg des Bindemittels, bestehend aus 99 Gew.-% Polyvinylacetat/Polyvinylpyrrolidon 50:50 und 1,0 Gew.-% hochdisperse Kieselsäure wurden unter Rühren in 900 ml gereinigtem Wasser dispergiert und langsam in den laufenden Diosna-Mischer eingetragen. Nach der Zugabe wurde noch 2 Minuten granuliert, die Konsistenz des Granulates geprüft und der Mischer entleert. Das feuchte Granulat wurde auf einer Horde bei 40°C abgetrocknet, wiederum in einem Diosna-Mischer gegeben, mit 0,45 kg gesiebtem Kollidon® CL und 0,05 kg gesiebtem Magnesiumstearat versetzt und 2 Minuten ohne Zerhacker gemischt.

Dieses Granulat wurde anschließend auf einer Korsch PH 106 Rundläuferpresse bei 18 kN Preßkraft zu biplanen, facettierten Tabletten mit 12 mm Durchmesser und 500 mg Gesamtgewicht verpreßt.

| Eigenschaften: | |
|---|---|
| Bruchfestigkeit: | 48 N |
| Friabilität: | 0,35 % |
| Zerfall: | 1 min |
| Freisetzung: (Methode nach USP) | 99,1 % nach 30 min |

Bei Verwendung eines Maltodextrins (DE-Wert 18) als Bindemittel in der gleichen Menge wurden lediglich 26 N und mit reinem Polyvinylpyrrolidon (K-Wert 30) 38 N erzielt.

**Patentansprüche**

**1.**    Verwendung von redispergierbaren Polymerpulvern oder Polymergranulaten aus

        a) 10 bis 95 Gew.-% Polyvinylacetat,

        b) 5 bis 90 Gew.-% eines N-Vinylpyrrolidon-haltigen Polymers,

        c) 0 bis 20 Gew.-% eines weiteren wasserlöslichen oder wasserquellbaren Stoffes und

        d) 0 bis 20 Gew.-% eines wasserunlöslichen Puderungsmittels sowie gegebenenfalls

        e) weiteren Zusatzstoffen

    als Bindemittel zur Herstellung von festen pharmazeutischen Darreichungsformen, wobei der Anteil des Bindemittels in der Darreichungsform 0,5 bis 20 Gew.-% beträgt.

**2.**    Verwendung von redispergierbaren Polymerpulvern oder Polymergranulaten nach Anspruch 1, die als Komponente b) ein Polymer aus der Gruppe Polyvinylpyrrolidon, Vinylacetat-Vinylpyrrolidon Copolymere oder Mischungen davon enthalten.

**3.**    Verwendung von redispergierbaren Polymerpulvern oder Polymergranulaten nach den Ansprüchen 1 und 2, die als Komponente c) ein Polymer aus der Gruppe Cellulosederivate, Acrylat-Methacrylat Copolymere und Polyvinylalkohole oder Mischungen davon enthalten.

**4.** Verwendung von redispergierbaren Polymerpulvern oder Polymergranulaten nach den Ansprüchen 1 bis 3, die ein wasserunlösliches Puderungsmittel aus der Gruppe Cellulose, disperse Kieselsäure, Talkum, Bentonit, Magnesiumstearat und Calciumphosphat oder Mischungen davon enthalten.

**5.** Verwendung von redispergierbaren Polymerpulvern oder Polymergranulaten nach den Ansprüchen 1 bis 4, **dadurch gekennzeichnet, daß** sie durch Direkttablettierung, Trockengranulation oder Feuchtgranulation mit dem Wirkstoff verarbeitet werden.

**6.** Feste pharmazeutische Darreichungsformen mit einem Bindemittel aus

a) 10 bis 95 Gew.-% Polyvinylacetat,

b) 5 bis 90 Gew.-% eines N-Vinylpyrrolidon-haltigen Polymers,

c) 0 bis 20 Gew.-% eines weiteren wasserlöslichen oder wasserquellbaren Stoffes und

d) 0 bis 20 Gew.-% eines wasserunlöslichen Puderungsmittels sowie gegebenenfalls

e) weiteren Zusatzstoffen,

wobei der Bindemittelanteil 0,5 bis 20 Gew.-% der Gesamtmenge ausmacht.

**7.** Feste pharmazeutische Darreichungsformen nach Anspruch 6, aus denen die Wirkstoffe innerhalb einer Zeit von 0,1 bis 1 Stunde freigesetzt werden.

**Claims**

**1.** The use of redispersible polymer powders or polymer granules consisting of

a) 10 - 95% by weight of polyvinyl acetate,

b) 5 - 90% by weight of an N-vinylpyrrolidone-containing polymer,

c) 0 - 20% by weight of another water-soluble or water-swellable substance and

d) 0 - 20% by weight of a water-insoluble dusting agent with or without

e) other additives,

as binders for producing solid pharmaceutical presentations, where the binder content in the presentation is from 0.5 to 20% by weight.

**2.** The use of redispersible polymer powders or polymer granules as claimed in claim 1 which comprise as component b) a polymer from the group of polyvinylpyrrolidone, vinyl acetate/vinylpyrrolidone copolymers and mixtures thereof.

**3.** The use of redispersible polymer powders or polymer granules as claimed in claims 1 and 2 which comprise as component c) a polymer from the group of cellulose derivatives, acrylate/methacrylate copolymers and polyvinyl alcohols or mixtures thereof.

**4.** The use of redispersible polymer powders or polymer granules as claimed in any of claims 1 to 3 which comprise a water-insoluble dusting agent from the group of cellulose, disperse silica, talc, bentonite, magnesium stearate and calcium phosphate or mixtures thereof.

**5.** The use of redispersible polymer powders or polymer granules as claimed in any of claims 1 to 4, wherein they are processed with the active ingredient by direct tableting, dry granulation or wet granulation.

**6.** A solid pharmaceutical presentation having a binder consisting of

a) 10 - 95% by weight of polyvinyl acetate,

b) 5 - 90% by weight of an N-vinylpyrrolidone-containing polymer,

c) 0 - 20% by weight of another water-soluble or water-swellable substance and

d) 0 - 20% by weight of a water-insoluble dusting agent with or without

e) other additives,

where the binder content is from 0.5 to 20% of the total weight.

**7.** A solid pharmaceutical presentation as claimed in claim 6, from which the active ingredients are released within a time of from 0.1 to 1 hour.

**Revendications**

**1.** Utilisation de poudres de polymères ou de granulés de polymères redispersables consistant en

a) 10 à 95 % en poids d'acétate de polyvinyle,
b) 5 à 90 % en poids d'un polymère contenant de la N-vinylpyrrolidone,
c) 0 à 20 % en poids d'une autre substance soluble ou gonflable dans l'eau et
d) 0 à 20 % en poids d'un agent de poudrage insoluble dans l'eau,
et le cas échéant,
e) d'autres additifs,

en tant que liants pour la préparation de formes d'administration pharmaceutiques solides, la proportion du liant dans la forme d'administration représentant 0,5 à 20 % en poids.

**2.** Utilisation de poudres de polymères ou de granulés de polymères redispersables selon la revendication 1, qui contiennent, en tant que composant b), un polymère du groupe de la polyvinylpyrrolidone, des copolymères acétate de vinyle-vinylpyrrolidone ou leurs mélanges.

**3.** Utilisation de poudres de polymères ou de granulés de polymères redispersables selon les revendications 1 et 2, contenant, en tant que composant c), un polymère du groupe des dérivés de la cellulose, des copolymères acrylate-méthacrylate et des alcools polyvinylique ou leurs mélanges.

**4.** Utilisation de poudres de polymères de granulés de polymères redispersables selon les revendications 1 à 3, contenant un agent de poudrage insoluble dans l'eau choisi parmi la cellulose, la silice dispersée, le talc, la bentonite, le stéarate de magnésium, le phosphate de calcium ou leurs mélanges.

**5.** Utilisation des poudres de polymères ou granulés de polymères redispersables selon les revendications 1 à 4, **caractérisée en ce qu'**on les façonne avec la substance active par mise directe en comprimés, granulation à sec ou granulation par voie humide.

**6.** Formes d'administration pharmaceutiques solides contenant un liant qui consiste en

a) 10 à 95 % en poids d'acétate de polyvinyle,
b) 5 à 90 % en poids d'un polymère contenant de la N-vinylpyrrolidone, ou
c) 0 à 20 % en poids d'une autre substance soluble ou gonflable à l'eau, et
d) 0 à 20 % en poids d'un agent de poudrage insoluble dans l'eau
et le cas échéant
e) d'autres additifs,

la proportion du liant représentant de 0,5 à 20 % du poids total.

**7.** Formes d'administration pharmaceutiques solides selon la revendication 6 à partir desquelles les substances actives sont libérées en une durée de 0,1 à 1 h.